# EUROPEAN PATENT APPLICATION

(11) **EP 3 306 336 A1**
(43) Date of publication of application: **11.04.2018**
(21) Application number: 16192761.1
(22) Date of filing: 07.10.2016
(51) Int. Cl.: G01R 33/48, G01R 33/3875, G01R 33/3873, A61N 5/10

(54) **HADRON THERAPY DEVICE AND MRI DEVICE HAVING MAGNETIC FIELD CORRECTING MEANS**

(71) Applicant: ION BEAM APPLICATIONS S.A., 1348 Louvain-la-Neuve (BE)
(72) Inventor: FORTON, Eric, 1348 Louvain-la-Neuve (BE); HENROTIN, Sébastien, 1348 Louvain-la-Neuve (BE)
(74) Representative: Pecher, Nicolas

(57) **Abstract**

Medical apparatus comprising:
-a magnetic resonance imaging (MRI) system for acquiring magnetic resonance data from an imaging zone (Vp), wherein the magnetic resonance imaging system comprises:
-a particle beam apparatus (1) having a particle beam line for producing, directing and managing a particle beam of charged particles,
-magnetic correcting means (31, 31a, 31b, 31c) for applying a magnetic correction to a magnetic field perturbation within the imaging volume (Vp)
-controlling means (33, 4) for controlling said magnetic correcting means (31, 31a, 31b, 31c),
wherein said controlling means are configured to provide a spatially optimized magnetic correction within a restricted volume of said imaging volume (Vp) during a spatially optimized magnetic correction period (Po), said spatially optimized magnetic correction period (Po) being posterior to said excitation period (Pe), wherein a selected slice (Vpi) from the imaging volume Vp is excited, and including at least one spatial encoding period (Pp, Pf) of the pulse sequence, said restricted volume comprising said selected slice (Vpi).

## Description

### Field of the invention

The invention relates to a medical apparatus combining a hadron therapy device with a magnetic resonance imaging device. The invention also relates to a method for correcting the magnetic perturbation within the imaging zone of a magnetic resonance imaging device.

### Description of prior artp

Document WO 2015/197475 discloses a medical apparatus combining a MRI device and charged particle beam apparatus for the real-time magnetic resonance imaging during the delivery of the charged particles beam. For charged particle beam therapy, real-time magnetic resonance imaging (MRI) during the delivery of the charged particle beam is challenging because of the strong magnetic fields associated with MRI and strong magnetic fields used for directing the particle beam to the target zone. Hence superposition of and interaction between the magnetic fields may cause severe problems, in particular for MRI. The disclosed apparatus includes an active compensation coil and a control unit, that attempt to cancel the stray field due to the bending magnet of the beam apparatus perturbating the longitudinal component of the MRI magnetic field within the MRI imaging volume. A perfect correction inside the sample is however not easily obtained and the current flowing in the active coils is computed thanks to a coil optimization program using a stream function method, which does not yield a solution to compensate the stray field inside the MRI imaging zone with a very high accuracy. Especially, the apparatus and method do not provide a correction sufficiently accurate for the spatial encoding of the excited atoms generating the MRI signal, this spatial encoding being strongly dependent on the local magnitude of the magnetic field in the MRI imaging volume.

### Summary of the invention

The present invention is defined in the appended independent claims. Preferred embodiments are defined in the dependent claims.

According to a first aspect, the invention relates to a medical apparatus comprising:
- a magnetic resonance imaging (MRI) system for acquiring magnetic resonance data from an imaging zone, wherein the magnetic resonance imaging system comprises:
   - a main magnetic unit for generating a uniform magnetic field within the imaging volume,
   - gradient coils and a RF unit for generating MRI pulse sequences, said MRI pulse sequences comprising an excitation step for exciting the spin of the nuclei of the excitable atoms in a selected slice of the imaging volume during an excitation period, said MRI pulse sequences also comprising spatial encoding steps of the excited atoms during spatial encoding periods;
   - antennas for receiving RF signals emitted by excited atoms;
- a particle beam apparatus having a particle beam line for producing, directing and managing a particle beam of charged particles,
- magnetic correcting means for applying a magnetic correction to a magnetic field perturbation within the imaging volume
- controlling means for controlling said magnetic correcting means,
wherein said controlling means are configured to provide a spatially optimized magnetic correction within a restricted volume of said imaging volume during a spatially optimized magnetic correction period, said spatially optimized magnetic correction period being posterior to said excitation period and including at least one spatial encoding period of the pulse sequence, said restricted volume comprising said selected slice.

By including at least one spatial encoding period of the pulse sequence, it is meant that said spatially optimized magnetic correction period is applied during the entire duration of said spatial encoding period.

In an advantageous embodiment, the restricted volume of said imaging volume is equal to the selected slice.

In another advantageous embodiment, the restricted volume of said imaging volume comprises the selected slice and a buffer zone around said selected slice.

In an advantageous embodiment, the controlling means are configured to provide an initial magnetic correction within the whole imaging volume during a time period preceding said spatially optimized magnetic correction period and including said excitation period. In this embodiment, the spatially optimized magnetic correction corresponds to an enhancement within said restricted volume of the initial magnetic correction.

In an advantageous embodiment, said particle beam apparatus comprises at least one bending magnet and at least one scanning magnet for directing the particle beam to an irradiation volume within the imaging volume, said at least one bending magnet and at least one scanning magnet being able to generate an adjustable magnetic field, said controlling means being configured to apply said magnetic correction according to the set point information of said at least one bending magnet and at least one scanning magnet.

In an advantageous embodiment, said particle beam apparatus comprises a gantry configured for rotating around a rotational axis, wherein the gantry comprises said at least one bending magnet, said controlling means being configured to adapt the magnetic correction according to the rotation angle of the gantry.

In an advantageous embodiment, said controlling means are configured to control the particle beam apparatus, the MRI system and the magnetic correcting means, said controlling means being able to receive in input the hadron therapy treatment plan and the MRI imaging treatment plan, said controlling means coordinating the execution of MRI pulse sequences, the delivery of the particle beam of charged particles and the magnetic correction applied by the magnetic correcting means.

In an advantageous embodiment, said magnetic correcting means comprise active coils, said active coils being arranged to allow a magnetic correction of the longitudinal and both transversal components of the magnetic field perturbation.

According to a second aspect, the invention relates to a method for correcting the magnetic perturbation within the imaging volume of a MRI apparatus, said MRI apparatus comprising:
- a main magnetic unit for generating a uniform magnetic field within the imaging volume,
- gradient coils and a RF unit for generating MRI pulse sequences, said MRI pulse sequences comprising an excitation step for exciting the spin of the nuclei of the excitable atoms in a selected slice of the imaging volume during an excitation period, said MRI pulse sequences also comprising spatial encoding steps of the excited atoms during spatial encoding periods;
- antennas for receiving RF signals emitted by excited atoms;
wherein said method comprises the step of providing a spatially optimized magnetic correction within a restricted volume of said imaging volume during a spatially optimized magnetic correction period, said spatially optimized magnetic correction period being posterior to said excitation period and including at least one spatial encoding period of the pulse sequence, said restricted volume comprising said selected slice.

### Short description of the drawings

These and further aspects of the invention will be explained in greater detail by way of example and with reference to the accompanying drawings in which:
**Figure 1** schematically shows two embodiments of a medical apparatus comprising a hadron therapy device coupled to a MRI;
**Figure 2** schematically shows a selection of an imaging slice, Vpi, in a MRI and creation of phase gradients and frequency gradients;
**Figure 3** schematically shows a typical MRI pulse sequence wherein a spatially optimized magnetic correction according to the invention is applied;
**Figure 4** schematically represents a first embodiment a control unit used in the apparatus according to the invention;
**Figure 5** schematically represents a second embodiment a control unit used in the apparatus according to the invention;
**Figure 6a), 6b) and 6c****)** illustrate two embodiments of the active coils used as magnetic correcting means in an apparatus according to the invention;
**Figure 7** represents an embodiment of magnetic correcting means comprising a passive ferromagnetic counterweight in an apparatus according to the invention;

The figures are not drawn to scale. Generally, identical components are denoted by the same reference numerals in the figures.

### Detailed description of preferred embodiments

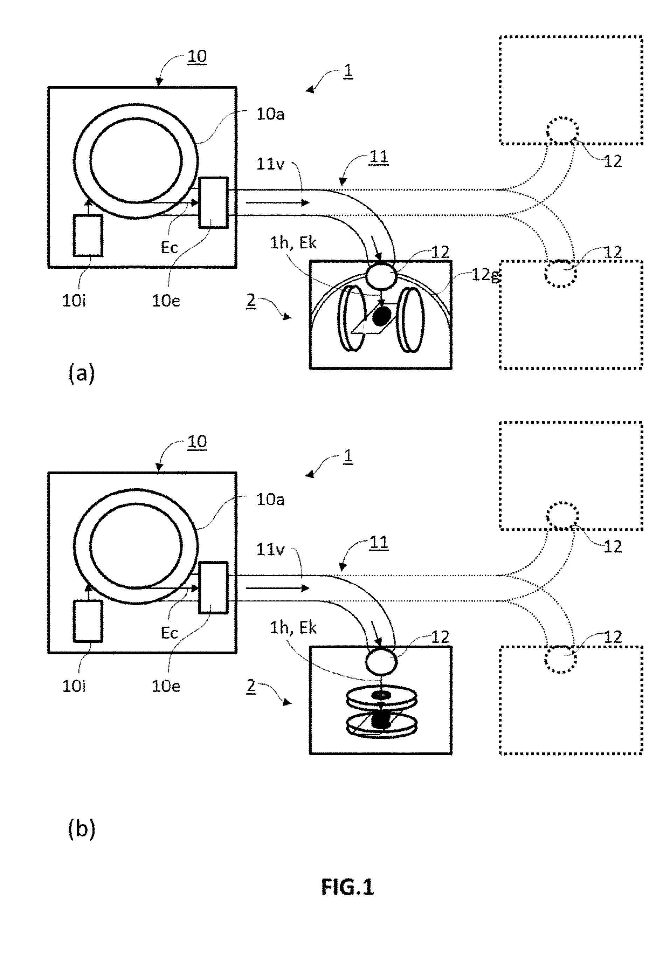

Figure 1 illustrates two examples of a medical apparatus comprising a hadron therapy device **1** coupled to a magnetic resonance imaging device (MRI) **2** according to the present invention. A hadron therapy device and a MRI and the combination of the two are described more in details in the following.

### Hadron therapy device

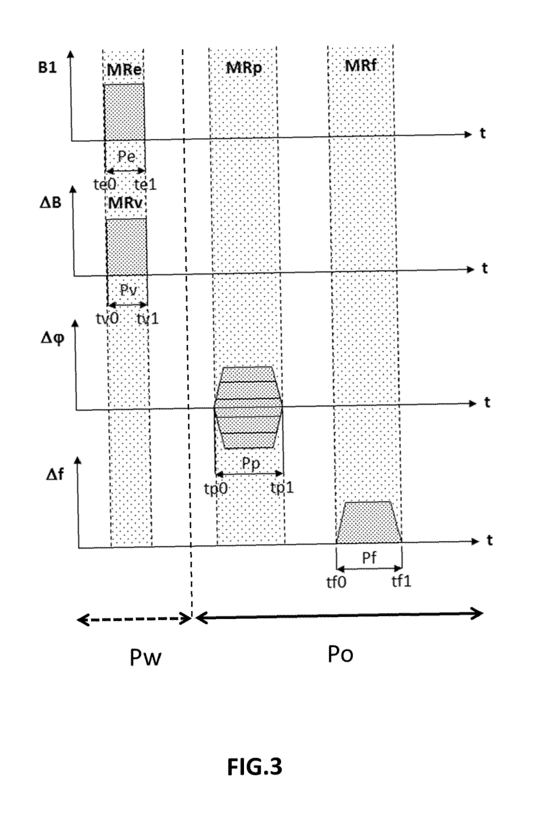

Hadron therapy is a form of external beam radiotherapy using beams **1h** of energetic hadrons. Figures 3, 4&6 show a hadron beam **1h** directed towards a target spot **40s** in a target tissue **40** of a subject of interest. Target tissues **40** of a subject of interest typically include cancerous cells forming a tumour. During a hadron therapy session, a hadron beam of initial energy, **Ek,** with k = 0 or 1, irradiates one or more target spots within the target tissue, such as a tumour, and destroys the cancerous cells included in the irradiated target spots, thus reducing the size of the treated tumour by necrosis of the irradiated tissues.

A hadron is a composite particle made of quarks held together by strong nuclear forces. Typical examples of hadrons include protons, neutrons, pions, and heavy ions, such as carbon ions. In hadron therapy, electrically charged hadrons are generally used. Preferably, the hadron is a proton and the corresponding hadron therapy is referred to as proton therapy. In the following, unless otherwise indicated, any reference to a proton beam or proton therapy applies to a hadron beam or hadron therapy in general.

A hadron therapy device **1** generally comprises a hadron source **10,** a beam transport line **11,** and a beam delivery system **12.** Charged hadrons may be generated from an injection system **10i**, and may be accelerated in a particle accelerator **10a** to build up energy. Suitable accelerators include for example, a cyclotron, a synchro-cyclotron, a synchrotron, or a laser accelerator. For example, a (synchro-)cyclotron can accelerate charged hadron particles from a central area of the (synchro-)cyclotron along an outward spiral path until they reach the desired output energy, **Ec,** whence they are extracted from the (synchro-)cyclotron. Said output energy, **Ec,** reached by a hadron beam when extracted from the (synchro-)cyclotron is typically comprised between 60 and 350 MeV, preferably between 210 and 250 MeV. The output energy, **Ec,** is not necessarily the initial energy, **Ek,** of the hadron beam used during a therapy session; **Ek** is equal to or lower than **Ec, Ek ≤ Ec.** An example of a suitable hadron therapy device includes, but is not limited to, a device described in US Pat. No. 8,410,730 or published U.S. Pat. App. No. 13/380,446, the entire disclosures of which are incorporated herein by reference as representative of suitable hadron beam therapy devices as used in the present invention.

The energy of a hadron beam extracted from a (synchro-)cyclotron can be decreased by energy selection means **10e** such as energy degraders, positioned along the beam path downstream of the (synchro-)cyclotron, which can decrease the output energy, **Ec,** down to any value of **Ek** including down to 0 MeV. As discussed supra, the position of the Bragg peak along a hadron beam path traversing specific tissues depends on the initial energy, **Ek,** of the hadron beam. By selecting the initial energy, **Ek,** of a hadron beam intersecting a target spot **40s** located within a target tissue, the position of the Bragg peak can be controlled to correspond to the position of the target spot.

As illustrated in Figure 3, downstream of the hadron source, a hadron beam of initial energy, **Ek,** is directed to the beam delivery system **12** through a beam transport line **11.** The beam transport line may comprise one or more vacuum ducts, **11v,** a plurality of magnets for controlling the direction of the hadron beam and/or for focusing the hadron beam, including the so-called bending magnet and scanning magnets. The beam transport line may also be adapted for distributing and/or selectively directing the hadron beam from a single hadron source **10** to a plurality of beam delivery systems for treating several patients in parallel.

The beam delivery system **12** comprises a nozzle for orienting a hadron beam **1h** along a beam path. The nozzle can either be fixed or mobile. Mobile nozzles are generally mounted on a gantry **12g.** A gantry is used for varying the orientation of the hadron outlet about a circle centred on an isocentre and normal to an axis, **Z,** which is generally horizontal. In supine hadron treatment devices, the horizontal axis, **Z,** may be selected parallel to a patient lying on a couch (i.e. the head and feet of the patient are aligned along the horizontal axis, **Z**). The nozzle and the isocentre define a path axis, **Xp,** which angular orientation depends on the angular position of the nozzle in the gantry. By means of magnets positioned adjacent to the nozzle, the so-called scanning magnets, the beam path of a hadron beam **1h** can be deviated with respect to the path axis, **Xp,** within a cone centred on the path axis and having the nozzle as apex. This is advantageous in that a volume of target tissue centred on the isocentre can be treated by hadron beam without changing the position of the nozzle within the gantry. The same applies to fixed nozzles with the difference that the angular position of the path axis is fixed.

### Magnetic resonance imaging device

A magnetic resonance imaging device **2** (MRI) implements a medical imaging technique based on the interactions of excitable atoms present in an organic tissue of a subject of interest with electromagnetic fields. When placed in a strong main magnetic field, **B0**, the spins of the nuclei of said excitable atoms precess around an axis aligned with the main magnetic field, **B0**, resulting in a net polarization at rest that is parallel to the main magnetic field, **B0**. The application of a pulse of radio frequency (RF) exciting magnetic field, **B1**, at the frequency of resonance, fL, called the Larmor frequency, of the excitable atoms in said main magnetic field, **B0**, excites said atoms by tipping the net polarization vector sideways (e.g., with a so-called 90° pulse, **B1-90)** or to angles greater than 90° and even reverse it at 180° (with a so-called 180° pulse, **B1-180).** When the RF electromagnetic pulse is turned off, the spins of the nuclei of the excitable atoms return progressively to an equilibrium state yielding the net polarization at rest. During relaxation, the transverse vector component of the spins produces an oscillating magnetic field inducing a signal which can be collected by antennas **2a** located in close proximity to the anatomy under examination.

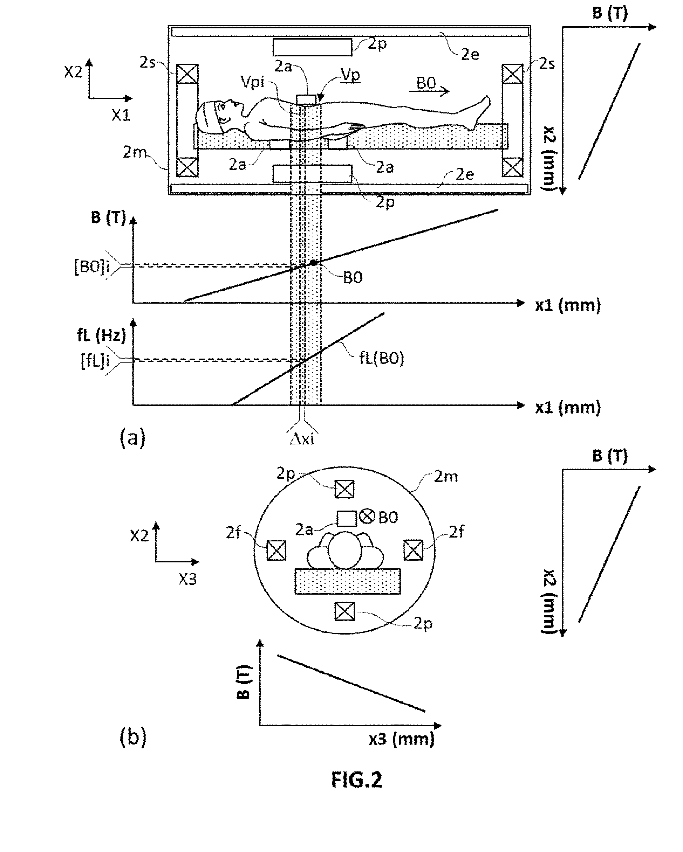

As shown in Figure 2, a MRI **2** usually comprises a main magnet unit **2m** for creating a uniform main magnetic field, **B0;** radiofrequency (RF) excitation coils **2e** for creating the RF-exciting magnetic field, **B1;** X1-, X2-, and X3-gradient coils, **2s, 2p, 2f,** for creating magnetic gradients along the first, second, and third directions **X1, X2,** and **X3,** respectively; and antennas **2a,** for receiving RF-signals emitted by excited atoms as they relax from their excited state back to their rest state. The main magnet produces the main magnetic field, **B0,** and can be a permanent magnet or an electro-magnet (a supra-conductive magnet or not). An example of a suitable MRI includes, but is not limited to, a device described in EP 0186238, the entire disclosure of which is incorporated herein by reference.

As illustrated in Figure 2, an imaging slice or layer, **Vpi,** of thickness, **Δxi**, normal to the first direction, **X1,** can be selected by creating a magnetic field gradient along the first direction, **X1.** In Figure 2, the first direction, **X1,** is parallel to the axis Z defined by the lying position of the patient, yielding slices normal to said axis Z. In practice, this is not necessarily the case, and the first direction, **X1,** can be any direction, e.g. transverse to the axis Z, with slices extending at an angle with respect to the patient. As shown in Figure 2, because the Larmor frequency, **fL,** of an excitable atom depends on the magnitude of the magnetic field it is exposed to, sending pulses of RF exciting magnetic field, **B1,** at a frequency range, **[fL]i,** excites exclusively the excitable atoms which are exposed to a magnetic field range, [**B0**]**i**, which are located in a slice or layer, **Vpi,** of thickness, **Δxi**. By varying the frequency bandwidth, **[fL]i,** of the pulses of RF exciting magnetic field, **B1**, the width, **Δxi**, and position of an imaging layer, **Vpi,** can be controlled. By repeating this operation on successive imaging layers, **Vpi,** an imaging volume, **Vp,** can be characterized and imaged.

To localize the spatial origin of the signals received by the antennas on a plane normal to the first direction, **X1,** magnetic gradients are created successively along second and third directions, **X2, X3,** wherein **X1** ⊥ **X2** ⊥ **X3,** by activating the X2-, and X3-gradient coils **2p, 2f,** as illustrated in Figure 2(b). Said gradients provoke a phase gradient, Δϕ, and a frequency gradient, **Δf**, in the spins of the excited nuclei as they relax, which allows spatial encoding of the received signals in the second and third directions, **X2, X3.** A two-dimensional matrix is thus acquired, producing *k*-space data, and an MR image is created by performing a two-dimensional inverse Fourier transform. Other modes of acquiring and creating an MR image are known in the art and the present invention is not restricted to the selection of any particular mode.

The MRI can be any of a closed-bore, open-bore, or wide-bore MRI type. A typical closed-bore MRI has a magnetic strength of 1.0 T through 3.0 T with a bore diameter of the order of 60 cm. An open-bore MRI has typically two main magnet poles **2m** separated by a gap between them for accommodating a patient in a lying position, sitting position, or any other position suitable for imaging an imaging volume, **Vp.** The magnetic field of an open-bore MRI is usually comprised between 0.2 and 1.0 T. A wide-bore MRI is a kind of closed-bore MRI having a larger diameter.

As described in Figure 3, a MRI device typically implements the following steps to acquire data from a sample to image:
- an **excitation step**(MRe) for exciting the spin of the nuclei of the excitable atoms, comprising creating an oscillating electromagnetic field, **B1**, with the RF unit at a RF-frequency range, **[fL]i,** corresponding to the Larmor frequencies of the excitable atoms exposed to antimagnetic field range, **[B0]i** = **[Bi,0, Bi,1]**, during an excitation period, Pe = (te1 - te0), wherein te0 is the time of the beginning of the oscillating electromagnetic field, and te1 is the end of the oscillating electromagnetic field;
- a **layer selection step** (MRv) for selecting a layer or slice, **Vpi,** of the imaging volume, **Vp,** of thickness, **Δxi**, measured along the first direction, X1, by creating a magnetic field gradient along the first direction, **X1,** of slope **dB** / **dx1** = **[B0]i / Δxi,**,
- a **phase gradient step**(MRp) for localising along the second direction, **X2,** the origin ofRF signals received by the antennas by varying a phase of the spins of the nuclei along the second direction, **X2,** and comprising the step of creating a magnetic field gradient along the second direction, **X2,** during a period, Pp = (tp1 - tp0), wherein tp0 is the time of the beginning of the phase gradient step, and tp1 is the end of the phase gradient step, with tp0 > te1; and
- a **frequency gradient step** (MRf) for localising along the third direction, **X3,** the origin of RF signals received by the antennas by varying a frequency of the spins of the nuclei along the third direction, **X3,** and comprising the step of creating a magnetic field gradient along the third direction, **X3,** during a period Pf= (tf1 - tf0), wherein tf0 is the time of the beginning of the frequency gradient step, and tfl is the end of the frequency gradient step, with tf0 > tp1;

The sequence of steps described above is just an example of a possible implementation of a MRI pulse sequence, which has to be repeated multiple times to image the successive layers of a sample, a layer having typically a thickness of 3mm. Other implementations, wherein the succession of excitation and gradient step is a bit different, can also be carried out. For example, the excitation step can be split into two steps wherein the first step is a 90° rotation of the longitudinal nuclear magnetization thanks to a first RF pulse, while second RF pulse is applied after the phase gradient step to cause a 180° rotation of transverse nuclear magnetization within the excited slice, in order to form a spin echo at subsequent time. In another implementation, the so-called MRI volumetric imaging, rather than selectively exciting a thin layer from the sample, a thick slice of about 10 cm to 20 cm is excited by a RF pulse. The spatial encoding of the excited atoms within the thick slice needs then consequently to be performed in three directions, typically with the help of two phase gradient steps and one frequency gradient steps. In the following, a (MRI) pulse sequence will refer to a succession of RF excitation steps and gradient steps allowing to implement any MRI technique as described in the prior art.

The medical apparatus according to the present invention comprises
- a magnetic resonance imaging (MRI) system for acquiring magnetic resonance data from an imaging zone Vp, wherein the magnetic resonance imaging system comprises:
   - a main magnetic unit 2m for generating a uniform magnetic field within the imaging volume Vp,
   - gradient coils 2s, 2p, 2f and a RF unit for generating MRI pulse sequences, said MRI pulse sequences comprising an excitation step MRe for exciting the spin of the nuclei of the excitable atoms in a selected slice Vpi of the imaging volume Vp during an excitation period Pe, said MRI pulse sequences also comprising spatial encoding steps MRp, MRf of the excited atoms during spatial encoding periods Pp, Pf;
   - antennas 2a for receiving RF signals emitted by excited atoms;
- a particle beam apparatus having a particle beam line for producing a particle beam of charged particles,
- magnetic correcting means for applying a magnetic correction to a magnetic field perturbation within the imaging volume Vp and controlling means for controlling said magnetic correcting means,
wherein said controlling means are configured to provide a spatially optimized magnetic correction within a restricted volume of said imaging volume Vp during a spatially optimized magnetic correction period Po, said spatially optimized magnetic correction period Po being posterior to said excitation period Pe and including at least one spatial encoding period Pp, Pf of the pulse sequence, said restricted volume comprising said selected slice Vpi.

As already explained above, the integration of MRI system and a particle beam apparatus and may cause severe problems, in particular for MRI, due to superposition and interaction between the different magnetic fields at stake. As already discussed in the prior art, the presence of magnetic correcting means is consequently essential in order to restore to some extent a magnetic field allowing performing the selective excitation and spatial encoding steps necessary to the implementation of the MRI imaging technique. In the present invention, the presence of controlling means allowing the spatial optimization of the magnetic correction during the MRI pulse sequence will however yield a higher accuracy in the spatial encoding steps. It is important to note that although the magnetic correcting means are advantageously used to correct the magnetic perturbation caused by said particle beam apparatus, they can also be used to apply a magnetic correction coming from other sources of perturbation in the environment of the medical apparatus.

It was indeed observed that once a slice Vpi of the imaging volume Vp has been selectively excited, the magnetic correction to perform the spatial encoding steps in this slice can be applied to a restricted volume of the imaging volume comprising the selected slice Vpi. This is because the magnitude of the magnetic field outside said selected slice Vpi will not harm the spatial encoding of the excited atoms within said selected slice Vpi and consequently it was observed that it is very advantageous to make full use of the magnetic correcting means to correct the magnetic field in a volume, the so-called restricted volume, which is spatially limited compared to the full imaging volume. The dedication of the magnetic correcting means to a magnetic correction within a spatially limited volume will indeed allow reaching a greater accuracy of the correction within such restricted volume. Such restricted volume can be chosen to be equal to the selected slice Vpi or alternatively to be a bit larger and to provide a buffer zone between the selected slice Vpi and the rest of the imaging volume. Such buffer zone will guarantee that the overlap between the targeted selected slice Vpi and the restricted volume with the spatially optimized correction is maximized.

As illustrated in Figure 3, the spatially optimized magnetic correction period Po is advantageously preceded by a period Pw during which a magnetic correction is applied to the whole imaging volume Vp. Such correction aims to allow an accurate slice selection step MRv within the imaging volume Vp, which implies that only the excitable atoms from said slice Vpi have their Larmor frequencies corresponding the frequency range [fL]iof the RF pulse generated by the RF unit. Such correction during a period Pw within the whole imaging volume Vp will be more spatially constraining for the magnetic correcting means than the spatially limited correction applied during the period Po, which will prevent it to be as accurate in terms of magnetic field magnitude. It was however observed that the accuracy of the magnetic correction is more critical during the spatial encoding steps than during the layer selection step MRv, this being the reason why this two levels magnetic correction is implemented in the medical apparatus according to the invention. If no magnetic correction is applied during the period preceding the spatially optimized magnetic correction period Po or if the correction applied during the period Pw is not sufficient to provide an accurate selective excitation of a layer having a regular slice shape Vpi, mathematical methods can be used to reconstruct a 3D MRI image based on the acquisition of RF signals generated by said excited irregular layer, the shape of said irregular layer being deducible from the knowledge of the magnetic perturbation within said imaging volume Vp.

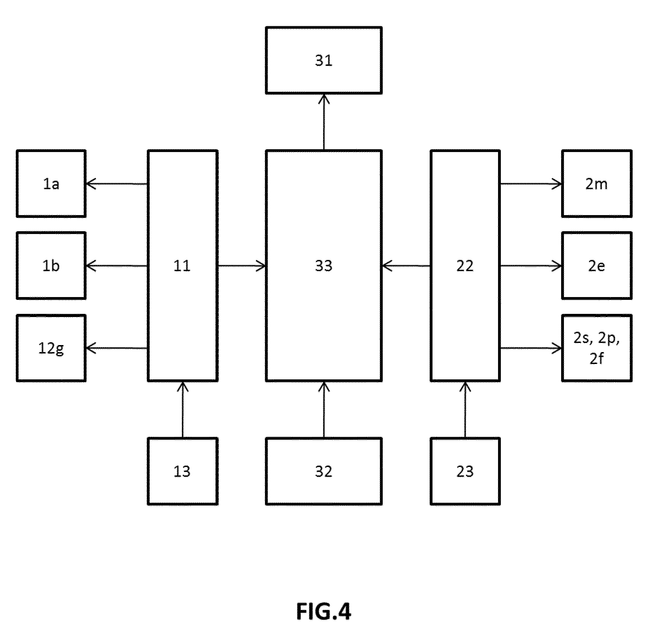

As illustrated in Fig. 4, the controlling means of the magnetic correcting means advantageously comprise a control unit **33** controlling active coils 31 used as magnetic correcting means. The control units receive data in input from both the control unit **11** of the hadron therapy device**1**, and the control unit **22** of the MRI device **2.** The control unit **11** of the hadron therapy device **1** is responsible for the execution of a treatment plan received from the hadron therapy user input device **13** and among other things exerts control over the bending magnet **1a** and the scanning magnets **1b**from the beam delivery system and also on rotation angle of the gantry **12g.** The set point information from said bending magnet **1a**, scanning magnets **1b** and gantry **12g** is delivered to the control unit **33.** The control unit **22** of the MRI on the other hand exerts control over the units generating the MRI pulse sequences, which include the excitation coils **2e,** the main magnetic unit **2m** and the gradient coil **2s**, **2p** and **2f.** The set point information from the units generating the MRI pulse sequences is also delivered by the MRI control unit **22** to the control unit **33.** The control unit **33** also receives from an input device **32**look-up tables of values of the correcting current to apply in the active coils in dependence of the currents flowing through the bending magnet**1a**, the scanning magnets **1b** and of the angle of rotation of the gantry **12g.** Such look-up tables can be obtained from simulations or experiments or a mix of both and will advantageously also make use of an optimization algorithm which will compute the currents in the active coil minimizing the magnetic field perturbation across the MRI imaging volume. As the control unit **33** receives in input data related to the MRI pulse sequences from the control unit **2,** it will be able to compute the spatially optimized magnetic correction within the restricted volume comprising selected slice Vpi and the corresponding period Po according to the invention, as detailed above.

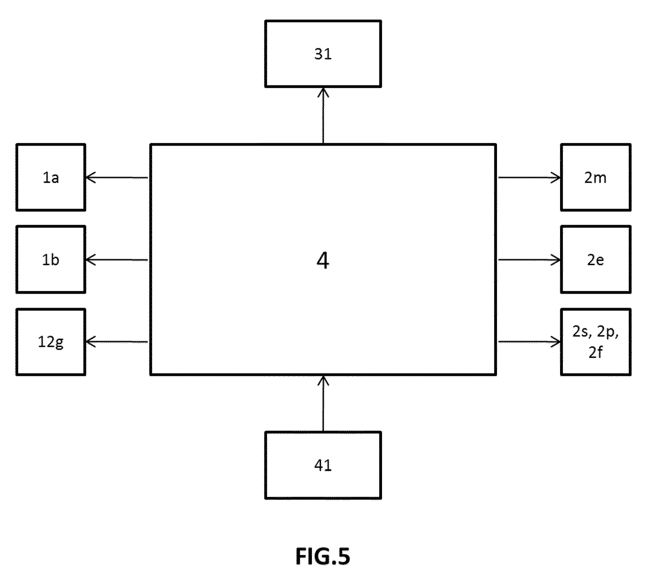

In an alternative advantageous embodiment illustrated in Figure 5, the controlling means comprise a central unit 4 controlling at the same time the active coils **31,** the various units of the hadron therapy device **1** and those of the MRI device **2.** In such an embodiment, this central unit **4** will receive from its input device **41a** broad range of information comprising the hadron therapy treatment plan, the MRI imaging plan and look-up tables of the correcting current in the active coils **31** described above, it will be able to optimize the global imaging and/or treatment sequence and then monitor both the hadron therapy treatment and the MRI imaging process to optimally coordinate the successive treatment steps, MRI pulse sequence steps and the magnetic correction delivered by the active coils **31.**

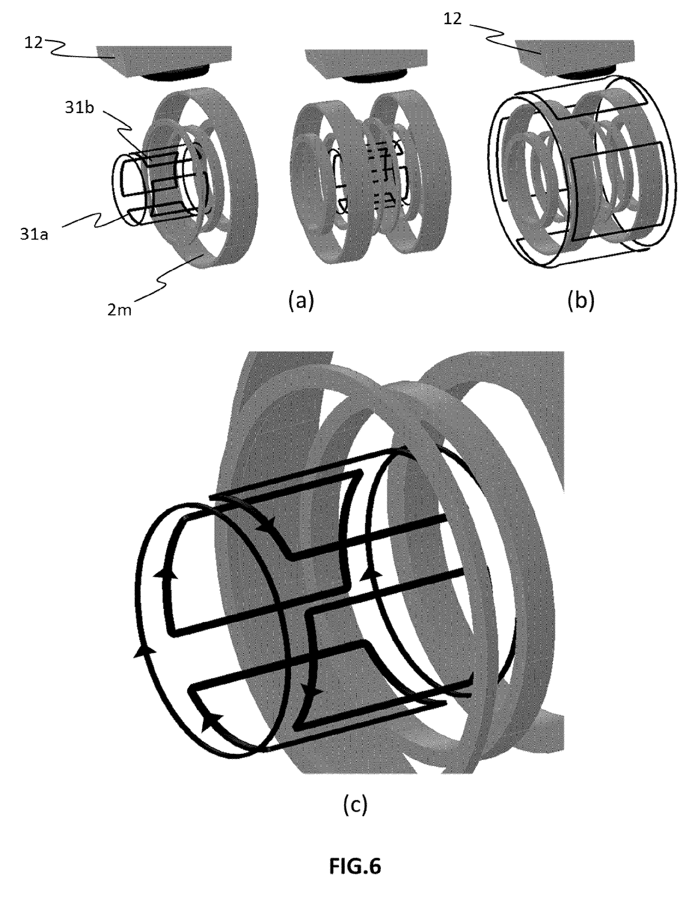

Figures 6a) and 6b) illustrate two embodiments of the active coils **31** used as magnetic correcting means. In Figure 6a), the active coils 31 comprise several current loops arranged in proximity to the inner surface of the MRI main magnetic unit 2m. The active coils 31 comprise one pair of lateral current loops 31a and several pairs of longitudinal current loops 31b. The presence of multiple current loops of various geometries 31a and 31b will allow correcting efficiently the magnetic perturbations inside the imaging volume of the MRI device. Especially, such coils will allow correcting the components of the magnetic field perturbation in the three spatial dimensions. In an advantageous embodiment, the current in each loop is driven independently in order to maximize the degrees of freedom of the magnetic correction. In Figure 6b), the active coils 31 are arranged in proximity to the outer surface of the MRI main magnetic unit 2m. The end of the beam delivery system **12** is also represented in these figures. Figure 6c) shows possible flowing directions for the current in the current loops from Figure 6a). It has to be noted that the magnetic correcting means according to the invention can comprise much more sophisticated coil geometries and with also a greater number of coils wherein the current is driven independently or not, in order to increase the number of degrees of freedom for increasing the accuracy of the spatially optimized magnetic correction according to the invention. Advantageously, the MRI gradient coils can also be used as magnetic correcting means, which means that the current flowing through these coils is chosen to compensate in some extent the magnetic perturbation generated inside the MRI imaging volume Vp, especially within excited slice Vpi.

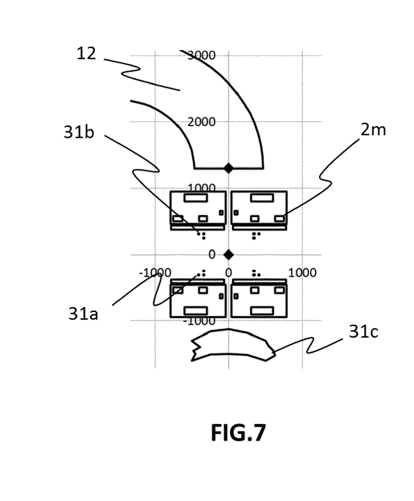

Figure 7 illustrates an embodiment of a passive ferromagnetic counterweight 31c used as a magnetic correcting means. Such passive ferromagnetic counterweight 31c is used as a complement to the active coils 31 and is designed to compensate the magnetic perturbation generated by the metallic structure of the gantry 12g. Such passive ferromagnetic counterweight 31c is advantageously fixed to the gantry 12g.

## Claims

1. Medical apparatus comprising:
- a magnetic resonance imaging (MRI) system for acquiring magnetic resonance data from an imaging zone (Vp), wherein the magnetic resonance imaging system comprises:
• a main magnetic unit (2m) for generating a uniform magnetic field within the imaging volume (Vp),
• gradient coils (2s, 2p, 2f) and a RF unit for generating MRI pulse sequences, said MRI pulse sequences comprising an excitation step (MRe) for exciting the spin of the nuclei of the excitable atoms in a selected slice (Vpi) of the imaging volume (Vp) during an excitation period (Pe), said MRI pulse sequences also comprising spatial encoding steps (MRp, MRf) of the excited atoms during spatial encoding periods (Pp, Pf);
• antennas (2a) for receiving RF signals emitted by excited atoms;
- a particle beam apparatus (1) having a particle beam line for producing, directing and managing a particle beam of charged particles,
- magnetic correcting means (31, 31a, 31b, 31c) for applying a magnetic correction to a magnetic field perturbation within the imaging volume (Vp)
- controlling means (33, 4) for controlling said magnetic correcting means (31, 31a, 31b, 31c),
**characterized in that** said controlling means are configured to provide a spatially optimized magnetic correction within a restricted volume of said imaging volume (Vp) during a spatially optimized magnetic correction period (Po), said spatially optimized magnetic correction period (Po) being posterior to said excitation period (Pe) and including at least one spatial encoding period (Pp, Pf) of the pulse sequence, said restricted volume comprising said selected slice (Vpi).

2. Medical apparatus according to claim 1 wherein the restricted volume of said imaging volume (Vp) is equal to the selected slice (Vpi).

3. Medical apparatus according to claim 1 wherein the restricted volume of said imaging volume (Vp) comprises the selected slice (Vpi) and a buffer zone around said selected slice (Vpi).

4. Medical apparatus according to anyone of the preceding claims wherein the controlling means are configured to provide a magnetic correction within the whole imaging volume (Vp) during a time period (Pw) preceding said spatially optimized magnetic correction period (Po) and including said excitation period (Pe).

5. Medical apparatus according to anyone of the preceding claims wherein said particle beam apparatus comprises at least one bending magnet (1a) and at least one scanning magnet (1b)for directing the particle beam to an irradiation volume within the imaging volume Vp, said at least one bending magnet (1a) and at least one scanning magnet (1b) being able to generate an adjustable magnetic field, said controlling means being configured to apply said magnetic correction according to the set point information of said bending magnet (1a) and at least one scanning magnet (1b).

6. Medical apparatus according to anyone of the preceding claims wherein said particle beam apparatus comprises a gantry (12g) configured for rotating around a rotational axis, wherein the gantry (12g) comprises said at least one bending magnet (1a), said controlling means being configured to adapt the magnetic correction according to the rotation angle of the gantry (12g).

7. Medical apparatus according to anyone of the preceding claims, wherein said controlling means (4) are configured to control the particle beam apparatus (1), the MRI system (2) and the magnetic correcting means(31, 31a, 31b, 31c), said controlling means (4) being able to receive in input the hadron therapy treatment plan and the MRI imaging treatment plan, said controlling means (4) coordinating the execution of MRI pulse sequences, the delivery of the particle beam of charged particles and the magnetic correction applied by the magnetic correcting means (31, 31a, 31b, 31c).

8. Medical apparatus according to anyone of the preceding claims, wherein said magnetic correcting means comprise active coils (31a, 31b), said active coils being arranged to allow a magnetic correction of the longitudinal and both transversal components of the magnetic field perturbation.

9. Method for correcting the magnetic perturbation within the imaging volume of a MRI apparatus, said MRI apparatus comprising
• a main magnetic unit (2m) for generating a uniform magnetic field within the imaging volume (Vp),
• gradient coils (2s, 2p, 2f) and a RF unit for generating MRI pulse sequences, said MRI pulse sequences comprising an excitation step (MRe) for exciting the spin of the nuclei of the excitable atoms in a selected slice (Vpi) of the imaging volume (Vp) during an excitation period (Pe), said MRI pulse sequences also comprising spatial encoding steps (MRp, MRf) of the excited atoms during spatial encoding periods (Pp, Pf);
• antennas (2a) for receiving RF signals emitted by excited atoms;
**characterized in that** it comprises the step of providing a spatially optimized magnetic correction within a restricted volume of said imaging volume (Vp) during a spatially optimized magnetic correction period (Po), said spatially optimized magnetic correction period (Po) being posterior to said excitation period (Pe) and including at least one spatial encoding period (Pp, Pf) of the pulse sequence, said restricted volume comprising said selected slice (Vpi).
